# FASCICULE DE BREVET EUROPEEN

(11) **EP 1 888 092 B1**
(45) Date de publication et mention de la délivrance du brevet: **22.10.2008**
(21) Numéro de dépôt: 06755503.7
(22) Date de dépôt: 18.05.2006
(51) Int. Cl.: A61K 36/02, A61P 7/08

(54) **MEDICAMENT A BASE DE LAMINARINE OU D' OLIGOLAMINARINES POUR LE TRAITEMENT DE LA SEPTICEMIE ET DU CHOC SEPTIQUE**
MEDIKAMENT AUS LAMINARIN ODER OLIGOLAMINARINEN ZUR BEHANDLUNG VON SEPTIKÄMIE UND SCHOCK
MEDICAMENT MADE FROM LAMINARINE OR OLIGOLAMINARINES FOR TREATMENT OF SEPTICEMIA AND SEPTIC SHOCK

(30) Priorité: 18.05.2005 FR 0504983
(43) Date de publication de la demande: 20.02.2008
(73) Titulaire: LABORATOIRES GOEMAR, 35400 Saint-Malo (FR)
(72) Inventeur: YVIN, Jean-Claude, F-35400 Saint-Malo (FR); DELZENNE, Nathalie, B-1332 Genval (BE)
(74) Mandataire: Cabinet Plasseraud
(86) Numéro de dépôt international: PCT/FR2006/001129
(87) Numéro de publication internationale: WO 2006/123059

(56) Documents cités:
- WO-A-02/36132
- ARRIETA S ET AL: "PHARMACOLOGICAL TREATMENT OF SEPTIC SHOCK" EXPERT OPINION ON THERAPEUTIC PATENTS, ASHLEY PUBLICATIONS, GB, vol. 10, no. 5, 2000, pages 601-622, XP001122150 ISSN: 1354-3776
- MIYANISHI N ET AL: "INDUCTION OF TNF-ALPHA PRODUCTION FROM HUMAN PERIPHERAL BLOOD MONOCYTES WITH BEAT-1,3-GLUCAN OLIGOMER PREPARED FROM LAMINARIN WITH BETA-1,3-GLUCANASE FROM BACILLUS CLAUSIL NM-1" JOURNAL OF BIOSCIENCE AND BIOENGINEERING, ELSEVIER, AMSTERDAM,, NL, vol. 95, no. 2, février 2003 (2003-02), pages 192-195, XP008043568 ISSN: 1389-1723

## Description

L'invention concerne un médicament à base de laminarine, ou d'oligolaminarines, propre au traitement de la septicémie et du choc septique.

La septicémie ou sepsis se caractérise par une réaction à l'infection excessive du système immunitaire et du système de coagulation de l'organisme. Cette réaction est marquée par une infection généralisée, c'est-à-dire de tout l'organisme, et par la coagulation du sang, phénomènes qui peuvent rapidement entraîner la défaillance d'un organe, il s'agit alors d'un sepsis sévère, et fréquemment le décès.

Le sepsis sévère se définit donc comme un sepsis accompagné de la défaillance d'un ou plusieurs organes comme, par exemple, les poumons, les reins, le système cardiovasculaire et/ou d'autres organes.

Le choc septique est une forme courante et grave de sepsis sévère. Il survient lorsque le système cardiovasculaire commence à défaillir et que la tension artérielle chute à un niveau dangereusement faible.

Les symptômes du sepsis peuvent varier d'une personne à l'autre, selon l'âge, la santé préalable et la réaction du patient à l'infection. L'évolution de la maladie est imprévisible, l'état de certains patients se détériorant rapidement pour passer au stade du sepsis sévère, ces derniers souffrant alors d'une défaillance des poumons, des reins, du système cardiovasculaire et/ou d'autres organes.

Le sepsis est généralement le résultat d'une infection bactérienne virale ou fongique qui peut survenir dans tout endroit de l'organisme.

Les personnes souffrant d'une pneumonie, d'un traumatisme, d'une intervention chirurgicale, de brûlures ou d'une maladie grave comme un cancer peuvent être prédisposées au sepsis ; souvent les personnes qui décèdent à la suite des complications d'un cancer ou d'une pneumonie décèdent en réalité d'un sepsis. Les personnes âgées et les patients dont le système immunitaire est affaibli par des médicaments, sont particulièrement vulnérables.

La mortalité peut atteindre 30 à 70% des cas.

Le nombre de cas de sepsis ou de choc septique causés par des bactéries gram+ ou gram-, des champignons, des virus et des parasites connaît une augmentation préoccupante probablement liée à
- l'augmentation de l'utilisation en milieu hospitalier de cathéters et autres équipements invasifs,
- l'augmentation de la chimiothérapie, ainsi que l'utilisation accrue d'immunosuppresseurs dans le cas de transplantations d'organes et
- l'accroissement de maladies inflammatoires graves.

Le sepsis et le choc septique sont traités par l'administration de
- fluides constitués par des sérums physiologiques enrichis en glucose.
- d'agents vasoconstricteurs pour restaurer la pression sanguine et
- d'antibiotiques.

Ces traitements comportant également l'oxygénation des patients.

Les traitements classiques dont il vient d'être question sont actuellement complétés par des approches alternatives visant à supprimer les effets délétères de l'inflammation et à stimuler les défenses anti-microbiennes, dont notamment l'administration
- d'anti-corps anti-LPS,
- d'anti-corps anti-TNF-α
- d'anticoagulants nouveaux, à savoir la protéine C active et la drotecogine

Le but de l'invention est, surtout, de donner au monde médical un moyen complémentaire de lutte contre la septicémie et le choc septique.

Or, il a été constaté que les phénomènes cliniques qui précèdent le développement de la septicémie ou du choc septique entraînent une très large activation des réponses cellulaires qui ont pour conséquence le relargage dans le sérum et dans le foie de toute une gamme de médiateurs inflammatoires tels que les cytokines et notamment la cytokine ou chimiokine TNF-α (Tumor Necrosis Faktor-α), les prostaglandines, les médiateurs lipidiques et les espèces activées de l'oxygène qui vont entretenir l'inflammation d'un certain nombre d'organes parmi lesquels les plus touchés sont les reins, le foie, les intestins et les poumons.

Le raisonnement qui est à la base de l'invention était donc, de trouver un moyen permettant de diminuer dans le sérum des malades la teneur en médiateurs inflammatoires rappelés ci-dessus et notamment en TNF-α et d'augmenter le nombre des cellules de Kupffer dont on connaît l'action notamment dans le renforcement de l'immunité et la lutte contre les pathogènes.

Et il est du mérite de la Société Demanderesse d'avoir trouvé que, de façon surprenante et inattendue, l'administration au malade, notamment par la voie orale, d'une quantité efficace de β-1,3-glucanes connus, s'agissant de la laminarine, notamment sous sa forme soluble et des oligolaminarines, permettait d'agir, dans le sens d'une diminution, sur la concentration en TNF-α et, dans le sens d'une augmentation, sur le nombre de cellules de Kupffer.

L'invention a donc pour objet l'utilisation de la laminarine, de préférence sous sa forme soluble ou d'oligolaminarines, pour la préparation d'un médicament destiné au traitement de la septicémie et du choc septique.

Selon un mode de réalisation avantageux, l'invention a pour objet l'utilisation de la laminarine, ou d'oligolaminarines, pour la préparation d'un médicament, pour l'administration par voie orale, destiné au traitement de la septicémie ou sepsis et du choc septique, ledit médicament étant administré au patient en une quantité suffisante pour apporter par jour à l'organisme une quantité de laminarine ou d'oligolaminarines, de 2 à 21 mg de préférence de 6 à 15 mg par kg de poids du corps.

Avant de donner la description des travaux qui ont permis à la Société Demanderesse de parvenir à l'invention, il est rappelé que par le terme laminarine on désigne des mélanges de polysaccharides du type β-1,3-glucane, extraits des algues brunes et dont le poids moléculaire est d'environ 2 500 à environ 6 000.

Les oligolaminarines sont, quant à elles un mélange d'oligo saccharides de type β-glucan issu de l'hydrolyse de la laminarine caractérisé par un degré de polymérisation entre 2 et 10, c'est à dire un poids moléculaire compris entre 350 et 2000.

La laminarine est constituée d'une chaîne principale linéaire de 15 à 35 unités glucopyranose réunies par des liaisons acétaliques β-1,3, cette chaîne principale portant une faible proportion de ramifications.

Le degré de polymérisation moyen est proche de 25.

La laminarine se présente sous une forme soluble et sous une forme insoluble.

Pour l'extraire des algues brunes et pour séparer en tant que de besoin les formes solubles et insolubles de la laminarine on peut se reporter notamment à la demande de brevet internationale WO 03/045 414 de la Société Demanderesse, publiée le 5 juin 2003.

La laminarine particulière qui a été utilisée dans le cadre des travaux décrits ci-après et qui ont permis de parvenir à l'invention, avait un poids moléculaire de 4500, un degré de polymérisation moyen de 25, et était consitituée essentiellement par des polysaccharides de degrés de polymérisation allant de 20 à 30.

Elle se présentait sous la forme d'une poudre blanc cassé, sans odeur et sans saveur.

Les travaux expérimentaux dont il a été question ci-dessus ont permis de montrer que la laminarine administrée notamment par voie orale exerçait un effet protecteur à l'égard des répercussions sur l'organisme d'un choc endotoxique.

En raison de ces résultats, on peut raisonnablement penser que la laminarine exerce un effet immuno-stimulant sur des cellules immunocompétentes telles que les macrophages et les cellules NK ("natural killer").

Dans le cadre des travaux en question,le choc endotoxique créé dans les organismes soumis aux expériences, a été provoqué par l'administration de lipopolysaccharides ou LPS dont l'effet s'apparente, comme connu, à une attaque bactérienne.

Les animaux sélectionnés pour les susdits travaux étaient des rats mâles Wistar Han (Harlem, Pays-Bas).

Les expériences ont été effectuées sur deux groupes de huit rats chacun.

Les rats de l'un des deux groupes - "groupe contrôle" avaient à leur disposition "ad libitum" une nourriture standard de marque AO4 (UAR, France) ; les rats de l'autre groupe avaient à leur disposition la même nourriture mais complétée en la susdite laminarine, commercialisée sous la marque "Dectinan" ; dans une première phase et pendant quatre jours (- 4 à - 1), la nourriture était enrichie à 5% en poids en Dectinan, puis à 10% en poids dans une deuxième phase de 21 jours (jours 0 à 21) ; ce deuxième groupe e rats est désigné par "groupe Dectinan".

C'est le 21^{ème} jour qu'a été provoqué sur les rats des deux groupes le choc endotoxique par injection intrapéritonéale de LPS en une quantité correspondant à 10mg par kg de poids du corps.

Aux jours 22 et 23, les rats ont été privés de nourriture et d'eau ; ils ont été sacrifiés au jour 24.

On a étudié les paramètres suivants :
- la consommation en eau et en nourriture
- l'évolution du poids corporel
- le bilan lipidique et glucidique
- la température rectale
- le nombre de globules blancs
- les médiateurs inflammatoires (PGE₂ ou prostaglandine E₂ et TNF-α sériques)
- les marqueurs histologiques des macrophages résidents du foie c'est-à-dire les cellules de Kupffer (ED-2 et/ou activité peroxydase)
- les marqueurs de toxicité du traitement LPS (activités ALT, AST et LDH sériques ; histologie du foie, de la rate, du thymus et des poumons)
- la fermentation caeco-colique (poids des fèces et prolifération caecale)

Dans le tableau I ci-après, on identifie les jours auxquels il est procédé aux différents prélèvements et mesures

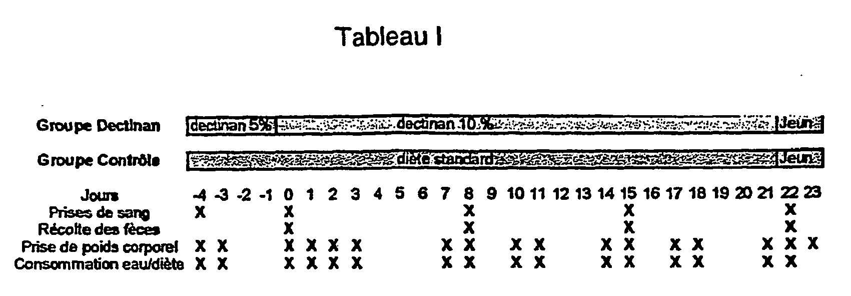

Plus particulièrement, on a procédé aux déterminations suivantes :
a) Avant l'injection de LPS
   - poids corporel
   - poids des fèces humides et après lyophilisation (récoltés durant 24 h)
   - poids de la diète et de l'eau de boisson
   - prise de sang de la veine caudale : dosage des triglycérides et du glucose
b) Après l'injection de LPS (aux heures 0-2-4-6-8 et 24)
   - température rectale
   - prise de sang dans la veine caudale : dosage des triglycérides, du glucose, du PGE₂ , du TNF-α, des acides gras libres, des activités ALT, AST et LDH (lactase deshydrogenase)
c) Au moment du sacrifice :
   - sang de la veine porte : dosage des acides gras libres
   - sang de la veine cave : détermination de la formule suaguine (comptage cellulaire, hématocrite et hémoglobine) et dosage des acides gras libres, des triglycérides, du PGE₂ , du TNF-α, des activités ALT, AST et LDH dans le sérum.
   - foie : poids, congélation de morceaux pour les études histologiques (hématoxyline-éosine, détection de cellules de Kupffer via l'activité peroxydase et l'antigène ED-2)
   - rate : poids et conservation de morceaux dans le formol pour les études histologiques (hématoxyline-éosine)
   - caecum : poids du caecum plein et du caecum vide
   - thymus et poumons : morceaux conservés dans le formol pour une étude histologique (hématoxyline-éosine)

Les résultats enregistrés ont été soumis à un traitement statistique.

A cet égard, les valeurs mentionnées plus loin représentent la moyenne ± ESM (Ecart Standart Moyen). Les résultats obtenus avant l'administration de LPS et au moment du sacrifice ont été analysés avec le test *t* de student (décrit dans : Appareils et Méthodes en Biochimie de Pierre Kamoun - 3ème édition - médecine sciences Flammarion 1987) entre les 2 groupes en utilisant le programme EXCELL ( décrit dans More Excell html +intro java +cd Auteur(s) GOTTLEBER - 01-2000). Les études cinétiques des différents paramètres mesurés après l'injection de LPS ont été traitées par une analyse ANOVA à 2 voies : analyse de variance, décrite dans « for the Behavorial Sciences Researcher » de Rudolph N. Cardinal publié, par les éditions LAWRENCE, via le programme SPSS (décrit dans Traitement de données avec SPSS pour Windows (édition 8.0, 9.0) Auteur(s) : OUELLET Femando, BAILLARGEON Gérald Date de parution: 01-2000.

Une transformation logarithmique des valeurs individuelles a été réalisée pour les paramètres TNF-α et PGE₂ avant le traitement statistique. Les différences sont considérées statistiquement significatives lorsque p<0.05 (p signifiant probabilité avec une tolérance de 5 %)

Les résultats obtenus avant l'administration de LPS sont comme indiqué ci-après :

Une semaine après leur arrivée, les rats avaient un poids moyen de 136 ± 1g et on été répartis de façon randomisée en 2 groupes de 8 animaux : le groupe contrôle et le groupe Dectinan ®. La prise de poids corporel ainsi que les consommations totales en eau et en nourriture du premier jour de traitement jusqu'au jour de l'injection de LPS sont indiquées dans le tableau 1. Le régime alimentaire n'a aucun effet sur ces 3 paramètres, ce qui est une preuve de l'absence de toxicité de la laminarine.

Les données enregistrées ont été réunies dans le tableau II ci-après :

**Tableau II. Prise de poids et consommation**

| | Contrôle | Dectinan® |
|---|---|---|
| Prise de poids (g) | 125 ± 3 | 125 ± 3 |
| Consommation totale en nourriture (g) | 449.5 ± 12.3 | 431.5 ± 6.0 |
| Consommation totale en eau de boisson (ml) | 576.9 ± 13.0 | 611.5 ± 17.9 |

| | | |
|---|---|---|
| n = 8. p > 0,05 test t de student | | |

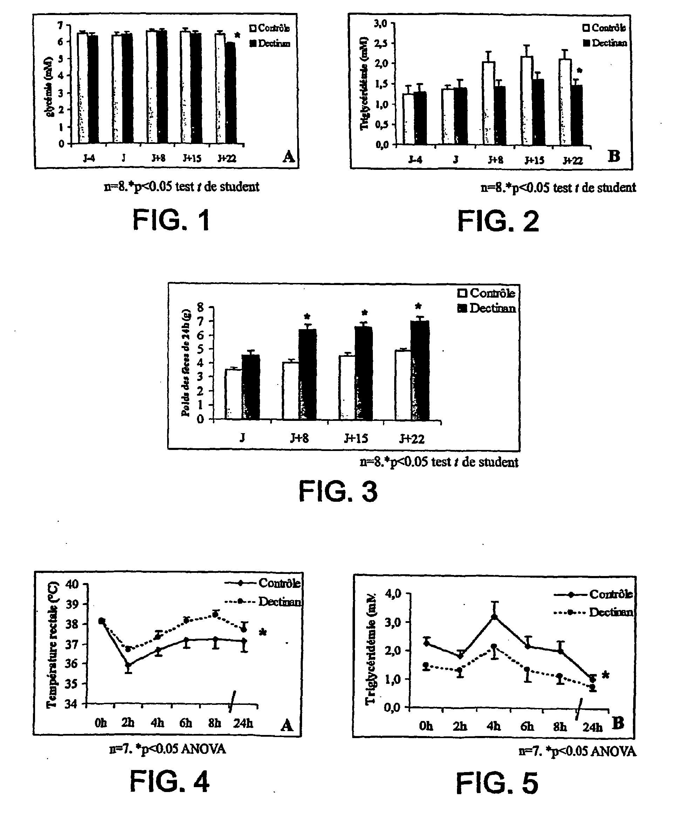

Des prises de sang hebdomadaires ont été effectuées afin de suivre l'évolution de la glycémie et de la triglycéridémie au cours du traitement alimentaire. Le régime enrichi en Dectinan® diminue signficativement la glycémie et la triglycéridémie au bout de 25 jours de traitement (4 jours à 5% et 21 jours à 10%) ainsi qu'il résulte respectivement des figures 1 et 2 qui illustrent l'évolution de la glycémie et de la triglycéridémie au cours du traitement

Les fèces de 24 heures ont été récoltées et pesées 1 fois par semaine.

Ainsi qu'il résulte du tableau montré à la figure 3, le régime enrichi en Dectinan® augmente la masse fécale dès la première semaine de traitement à 10%.

La perte d'eau dans les fèces est également plus importante ; à la troisième semaine d'administration de nourriture enrichie en Dectinan® à 10%, le pourcentage d'eau des fèces est de 24.7 ± 1.4% alors qu'il est de 17.8 ± 1.5% pour le groupe contrôle (*p<0.05, test *t* de student).,

Le LPS induit une hypothermie et une hyperglycémie transitoires 2 heures après son administration. Le régime enrichi en Dectinan® amplifie ces deux phénomènes de manière significative. En outre, les rats du groupe Dectinan maintiennent une température supérieure aux rats du groupe contrôle jusqu'au moment du sacrifice.

Le traitement préalable pendant 25 jours avec le Dectinan® mélangé à la nourriture empêche, ou pour le moins aménuise, les effets métaboliques résultant de l'inflammation aiguë générée par le LPS. Autrement dit le Dectinan® aménuise l'hypothermie, l'hypertriglycéridémie et l'hyperglycémie des premières heures qui suivent l'injection de LPS ainsi qu'il résulte des courbes apparaissant sur les graphiques des figures 4 (hypothermie), 5 (hypertriglycéridémie) et 6 (hyperglycémie).

En d'autres termes, l'administration d'une nourriture enrichie en Dectinan® protège le foie contre les effets toxiques liés à l'inflammation aiguë, mimée par l'injection au LPS, un phénomène qui va de pair avec une modulation des cellules immunitaires du foie.

Par ailleurs, l'administration de cette nourriture agit contre l'augmentation du marqueur spécifique d'une souffrance hépatique, ce marqueur étant constitué par l'activité sérique de l'alanine aminotransférase (ALT) ; elle corrige également d'autres paramètres sériques qui peuvent refléter les altératioris-tissulaires dans plusieurs organes, dont le foie, à savoir les activités aspartate aminotransférase (AST) et lactate déhydrogénase (LDH).

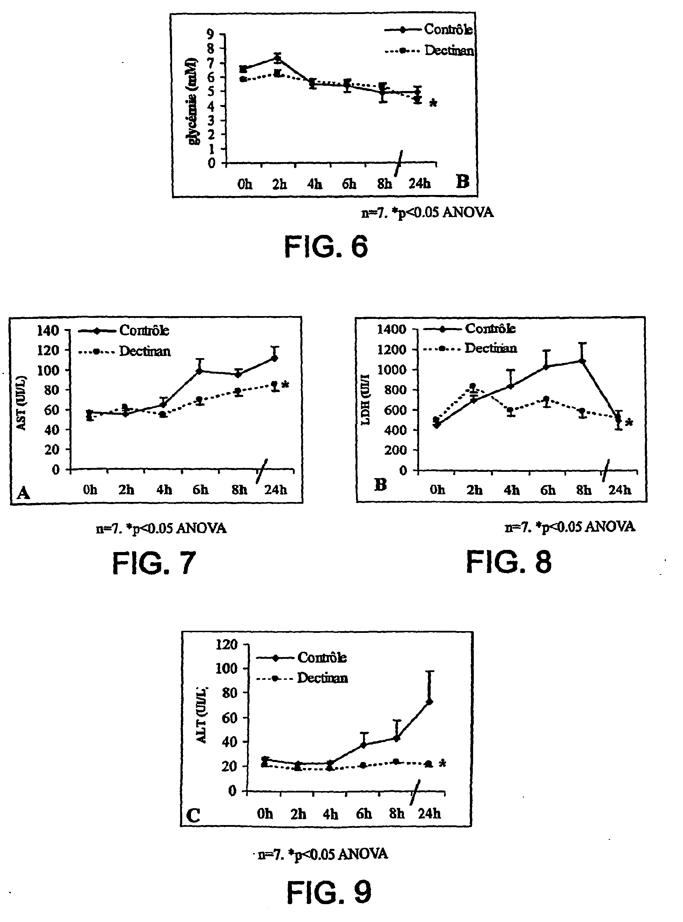

Les résultats enregistrés se traduisent par les courbes montrées aux figures 7 (évolution de l'AST), 8 (évolution du LDH) et 9 (évolution de l'ALT).

On a également mesuré la concentration dans le sérum (en pg/ml)
- de la cytokine TNF-α aux temps 2 heures, 4 heures, 6 heures, 8 heures et 24 heures après l'administration de LPS.
- de la prostaglandine PGE2 aux temps 2 heures et 24 heures après l'administration de LPS.

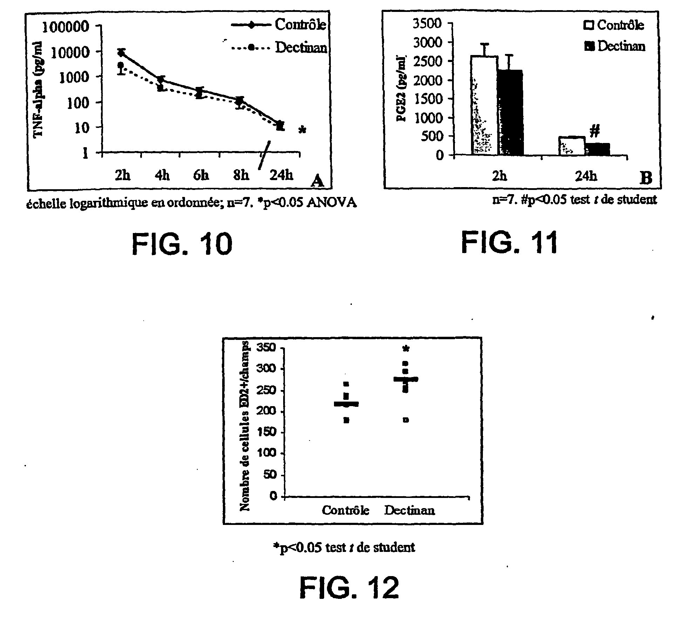

Les résultats sont reportés respectivement sur les graphiques des figures 10 (TNF-α) et 11 (PGE2).

On note que le TNF-α n'est pas détectable au temps 0 heure en raison de l'absence de stimulus inflammatoire. On note par ailleurs que la présence de Dectinan dans la nourriture diminue le taux de TNF-α et de PGE₂ dans le sérum des rats soumis au choc endotoxique.

Les rats ont été mis à jeun après l'administration de LPS afin d'éviter des problèmes d'interprétation liés à l'effet anorexigène de ce traitement.

Après le sacrifice, on a procédé aux différentes pesées dont les résultats sont réunis dans le Tableau III. ≠

**Tableau III. Poids corporel et poids des organes**

| | Contrôle | Dectinan® |
|---|---|---|
| Poids corporel (g) | 239 ± 4 | 234 ± 3 |
| Poids des organes corporels (%) | | |
| - foie | 3,56 ± 0,13 | 3,88 ± 0,08≠ |
| - rate | 0,36 ± 0,02 | 0,35 ± 0,02 |
| -caecum vide | 0,31 ± 0,02 | 0,47 ± 0,01* |

| | | |
|---|---|---|
| n = 7. ≠p = 0,07 ; *p< 0,05 test t de student | | |

A l'examen des résultats réunis dans le Tableau III, on peut faire les constatations suivantes.

La perte de poids n'est pas significativement modifiée par l'administration de la nourriture au Dectinan ® (25 ± 2g *versus* 22 ± 1g ; rapporté au poids corporel). Le poids des animaux n'est pas différent entre les 2 groupes après 24 heures de jeûne. Le poids du foie augmente légèrement chez le groupe Dectinan® avec une valeur p proche de la signification. Le régime enrichi en Dectinan® induit une prolifération caecale importante comme en attestent les valeurs moyennes du poids du tissu caecal et du contenu caecal (3.2 ± 0.32g pour le groupe Dectinan® *versus* 2.15 ± 0.24 g pour contrôles* p<0.05 test *t* de student).

Il a également été procédé au comptage des globules rouges et blancs ainsi qu'aux mesures de différents paramètres sanguins qui ont été réalisées dans les prélèvements de sang au niveau de la veine cave.

Les résultats sont réunis dans le Tableau IV.

**Tableau IV. Formule sanguine**

| | Contrôle | Dectinan® |
|---|---|---|
| Globules blancs (x 1000/mm³) : | 3,59 ± 0,14 | 4,02 ± 0,28 |
| - monocytes (%) | 2,74 ± 0,17 | 2,14 ± 0,19* |
| - lymphocytes (%) | 33,12 ± 2,23 | 31,67 ± 2,42 |
| -granulocytes (%) | 64,15 + 2,13 | 66,18 ± 2,39 |
| Globules rouges (x 1000/ mm³) | 7,38 ± 0,13 | 7,31 ± 0,20 |
| Hématocrite (%) | 43,7 ± 1,0 | 42,6 ± 1,0 |
| Hémoglobine (g/dl) | 13,6 ± 0,3 | 13,3 ± 0,3 |

| | | |
|---|---|---|
| n = 7.*p< 0.05 test t de student | | |

A l'examen des résultats réunis dans le Tableau IV, on constate que seul le pourcentage de monocytes parmi les globules blancs est significativement différent ; il est plus faible chez les rats nourris avec un régime enrichi en Dectinan® par rapport aux rats du groupe contrôle.

La détection de l'antigène membranaire ED2 par une technique immuno-histochimique permet la détection spécifique des cellules de Kupffer dans le foie. Ces cellules se situent surtout au niveau des espaces portes ; elles sont plus rondes et gonflées 24 heures après l'administration de LPS. Afin d'affiner l'observation des coupes de foie, une approche quantitative a été réalisée : 3 champs de 10 x 10 carrés-contenant au moins 3 vaisseaux appartenant à un espace porte- ont été sélectionnés par coupe de foie afin de réaliser un comptage des cellules ED2- positives. Le prétraitement avec une diète enrichie en Dectinan® augmente le nombre de macrophages hépatiques (259 + 19 cellules ED2 +/ champ *versus* 217 ± 14 cellules ED2 +/champ, n=6; p= 0.1, test t de student) ; ces macrophages résidents du foie épurent le foie des lipopolysaccharides circulants. Quand la valeur du rat 8 (représentée par un carré blanc) anormalement basse, appartenant au groupe Dectinan® n'est pas reprise dans la moyenne, la différence est significative.

Le résultat des susdites mesures est illustré par la figure 12 qui montre la quantification des cellules de Kupffer par le moyen de la détection de ED2 dans les coupes de foie

On sait que l'enzyme peroxydase est présente au sein des macrophages (tels que les cellules de Kupffer), des neutrophiles et des éosinophiles. L'administration de LPS augmente le nombre de cellules positives dans le foie car des cellules immunitaires (neutrophiles) sont recrutées. Le régime supplémenté en Dectinan® semble diminuer le nombre de cellules positives.

L'analyse histologique après coloration à l'éosine et à l'hématoxyline révèle l'architecture des tissus ainsi que les cellules en souffrance ou les foyers de nécrose. Au niveau du foie les altérations, principalement des foyers nécrotiques, liées à l'injection de LPS ne sont pratiquement pas visibles, tant au niveau du groupe Dectinan® qu'au niveau du groupe contrôle ; on note que les altérations biochimiques (principalement l'augmentation de l'ALT) sont en général plus précoces et ne se traduisent pas nécessairement par des altérations au niveau du tissu hépatique. Le poumon présente des oedèmes typiquement liés à l'état inflammatoire tandis que le thymus et la rate révèlent des lésions nécrotiques

L'ensemble des résultats qui précèdent traduit l'effet protecteur du Dectinan® vis-à-vis de l'augmentation des activités AST, LDH et ALT dans le sérum. On observe que la toxicité du LPS est moindre en raison de l'effet protecteur ; elle est corrélée à une moindre sécrétion de TNF-α qui est une cytokine proinflammatoire. On observe également une diminution des concentrations de PGE₂, vingt-quatre heures après le stimulus inflammatoire.

L'hypothermie constitue une manifestation précoce après l'administration d'une dose importante de LPS. Le TNF-α est en fait considéré comme signal "cryogénique" impliqué dans le maintien d'une hypothermie ; ce phénomène est plus prononcé chez les rats du groupe contrôle qui ont, en l'occurrence des taux de TNF-α plus élevés que ceux du groupe Dectinan®.

Le choc endotoxique réalisé par l'administration de LPS produit également une hyperglycémie transitoire, due en partie à une glycogénolyse hépatique intense et suivie par une diminution progressive de la glycémie, résultant de l'utilisation du glucose par les tissus périphériques et en particulier par les cellules immunitaires. L'hyperglycémie transitoire étant moins importante chez les rats du groupe Dectinan®., la glycogénolyse hépatique pourrait être évoquée comme cible métabolique potentielle.

L'administration de fortes doses de LPS induit une hypertriglycéridémie que l'on retrouve dans les deux groupes d'animaux. Les taux de triglycérides sériques sont systématiquement inférieurs après une diète enrichie en Dectinan® mais les variations de triglycérides due à l'injection de LPS sont similaires. Une baisse des taux de triglycérides dans le sang, en dehors du stimulus inflammatoire, constitue un résultat très intéressant.

L'étude histologique de l'antigène ED2 met en évidence une augmentation du nombre des cellules de Kupffer sous le régime enrichi en Dectinan® ; il pourrait s'agir soit d'un recrutement de cellules souches issues de la moëlle, soit d'une prolifération intra-hépatique de cellules de Kupffer existantes. Quant à l'étude histologique de la peroxydase endogène, qui résulte de l'activité des cellules de Kupffer et des cellules immunitaires recrutées lors d'un état inflammatoire, elle semble être moins importante après une diète enrichie en Dectinan®. Par ailleurs, il est intéressant de constater que le nombre de monocytes circulants est moins élevé dans le groupe Dectinan® que dans le groupe contrôle après l'administration de LPS.

En conclusion, il est intéressant de constater qu'une administration de nourriture enrichie en Dectinan® permet de diminuer l'inflammation liée au LPS, avec des conséquences favorables sur les enzymes d'origine hépatique, et sur le recrutement du nombre de cellules immunitaires dans le foie, ce phénomène allant de pair avec une augmentation du nombre de macrophages hépatiques.

Le médicament obtenu par l'utilisation conforme à l'invention de la laminarine ou d' oligolaminarines peut se présenter sous forme de comprimés, de gélules, de sachets ou encore par exemple sous une forme liquide, notamment d'une solution buvable.

### Exemple 1 : Comprimés

Comprimés nus de 450mg à base de Dectinan® :

Leur formule est comme suit :
- 225 mg de Dectinan® soit 50%
- 176 mg de Maltodextrines soit 39.1 %
- 40 mg de phosphate tricalcique soit 8,9 %
- 9mg de stéarate de magnésium soit 2%

Les constituants sont tamisés, mélangés puis comprimés sous forme de lingots. Ceux-ci sont ensuite introduits dans un broyeur qui les transforme en granulés de taille convenant à la phase de compression du comprimé final.

Les comprimés peuvent être commercialisés sous forme nue, pelliculée ou dragéifiée. Ils peuvent être conditionnés en pilulier ou en plaquettes thermoformées de 15 ou 20 unités.

La posologie est de 2 à 4 comprimés par jour, en 2 prises au cours des repas ; à consommer par cures de 15 jours.

### Exemple 2 : Gélules

Gélules d'origine végétale ou marine de 363 mg à base de Dectinan® :

Leur formule est comme suit pour 288 mg de poudre :
- 225 mg de Dectinan® soit 78.1 %
- 60 mg de Cellulose microcristalline soit 20.8%
- 2 mg de silice soit 0.7%
- 1 mg de stéarate de magnésium soit 0.4%

Ces gélules peuvent être conditionnées en pilulier ou en plaquettes thermoformées de 15 ou 20 unités.

La posologie est de 2 à 4 gélules par jour, en 2 prises au cours des repas : à consommer par cures de 15 jours

### Exemple 3 : Sachets

Sachets doses de 1g à base de Dectinan® :

Leur formule est comme suit :
- 225 mg de Dectinan® soit 22.5%
- 275 mg de maltodextrines soit 27.5 %
- 295mg de fructose soit 29.5%
- 200 mg de poudre de jus de citron soit 20%
- 5 mg de silice soit 0.5 %

Un litre d'une solution aqueuse contenant 22.5 g de Dectinan® est mélangée avec 27.5g de maltodextrines. Puis les autres constituants sont incorporés. La solution ainsi obtenue est ensuite atomisée puis la poudre granulée. La poudre est ensuite séchée à 60°C pendant une nuit de manière à obtenir une matière sèche inférieure à 3%. Le conditionnement est réalisé ensuite dans des sachets en aluminium, de taille 60 x 30mm contenant 1 g de poudre.

La posologie est de 2 à 4 sachets par jour, en 2 prises au cours des repas ; à consommer par cures de 15 jours.

### Exemple 4 : Solution buvable

- Eau soit 92.85%
- Dectinan® soit 2.25 %
- Fructose soit 4%
- Arôme citron soit 0.2%
- Acide citrique soit 0.5%
- Sorbate de potassiuùm soit 0.1 %
- Benzoate de sodium soit 0.1 %

Cette solution buvable peut être conditionnée en unidoses de 10 ml ou en flacon de 100 ml.

La posologie est de 2 à 4 doses de 10 ml ou 2 à 4 cuillères à soupe par jour, en 2 prises au cours des repas ; à consommer par cures de 15 jours.

## Revendications

1. Utilisation de la laminarine, de préférence sous sa forme soluble ou d'oligolaminarines, pour la préparation d'un médicament destiné au traitement de la septicémie et du choc septique.

2. Utilisation de la laminarine, ou d'oligolaminarines, pour la préparation d'un médicament pour l'administration par voie orale destiné au traitement de la septicémie et du choc septique, ledit médicament étant administré au patient en une quantité suffisante pour apporter par jour à l'organisme une quantité de laminarine ou d'oligolaminarines, de 2 à 21 mg de préférence de 6 à 15 mg par kg de poids du corps.

## Claims

1. Use of laminarin, preferably under its soluble form, or of oligolaminarins, for the manufacture of a medicament for the treatment of septicemia and of septic shock.

2. Use of laminarin, or of oligolaminarins, for the manufacture of a medicament for oral administration for the treatment of septicemia and of septic shock, wherein said medicament is administered to the patient in a sufficient quantity to provide per day to the organism a quantity of laminarin or of oligolaminarins of 2 to 21 mg, preferably of 6 to 15 mg per kg of body weight.

## Patentansprüche

1. Verwendung von Laminarin, vorzugsweise in seiner löslichen Form, oder von Oligolaminarinen für die Zubereitung eines zur Behandlung der Septikämie oder des septischen Schocks bestimmten Medikaments.

2. Verwendung von Laminarin oder von Oligolaminarinen für die Zubereitung eines zur Behandlung der Septikämie oder des septischen Schocks bestimmten Medikaments zur Oralverabreichung, wobei das genannte Medikament dem Patienten in einer Menge verabreicht wird, die ausreicht, um pro Tag dem Organismus eine Menge von Laminarin oder Oligolaminarinen von 2 bis 21 mg, vorzugsweise von 6 bis 15 mg, pro kg Körpergewicht zuzuführen.
